# EUROPEAN PATENT APPLICATION

(11) **EP 4 257 142 A1**
(43) Date of publication of application: **11.10.2023**
(21) Application number: 22167271.0
(22) Date of filing: 08.04.2022
(51) Int. Cl.: A61K 35/12, A61K 35/54, A61P 1/16, A61K 35/545

(54) **THE METHOD OF TREATMENT OF CHRONIC DIFFUSE LIVER LESIONS BY INTRAVENOUS TRANSPLANTATION OF STEM CELLS**

(71) Applicant: Saparbayev, Samat S., 010000 Nur-Sultan (KZ); Kurakbayev, Yedil Bekbayevich, 050000 Almaty (KZ); AL-Zhami TOO, 010000 Nur-Sultan (KZ)
(72) Inventor: SAPARBAYEV, Samat S., 010000 Nur-Sultan (KZ); KURAKBAYEV, Yedil Bekbayevich, 050000 Almaty (KZ)
(74) Representative: Jeck, Jonathan

(57) **Abstract**

The treatment refers to the field of clinical medicine, namely, transplantology, cell therapy and biotechnology.

The method of intravenous transplantation of stem cells (hepatocytes) is distinguished by the fact that it eliminates surgical intervention and excludes further complications from invasion.

The introduction of this method will prevent psycho-anatomical traumatization of the human body. Reduce the frequency of liver failure progression after intravenous stem hepatocyte transplantation. Ability to live without immunosuppressive therapy. Improve the functional quality of the liver. Improve the general condition of a patient with chronic diffuse liver damage. Improve the prognosis of the course of the disease and enable a five-year survival rate. Cell therapy can be considered as an independent method of treatment of patients with chronic diffuse liver lesions.

## Description

The AI-Zhami LLP (founder Tolegenova A.S.), Saparbayev S.S., Kurakbayev Ye.B.

The invention belongs to the field of clinical medicine, in particular to transplantology, cell therapy and biotechnology and can be used in the treatment of chronic diffuse liver diseases (CDLD).

### Relevance

Global mortality rates due to liver disease are approximately two million, annually. This includes one million deaths caused by complications due to cirrhosis and the remaining owing to viral hepatitis and liver cancer. The global prevalence of viral hepatitis remains high, while drug-induced liver injury continues to increase as a major cause of acute hepatitis. Liver transplantation is the second most common solid organ transplantation, yet less than 10% of global transplantation needs are met at current rates.

Organ transplantation is predicted to increase as life expectancy and the incidence of chronic diseases rises. Regenerative medicine-inspired technologies challenge the efficacy of the current allograft transplantation model.

The direction of clinical transplantation - cell transplantation - allows to avoid many disadvantages of whole liver transplantation. There is no need for traumatic and voluminous surgical intervention, the need for donor material is significantly reduced. At the same time, fetal stem cells are the most optimal for transplantation due to the lack of expression of histone compatibility proteins, the ability to integrate into the liver tissue of the recipient, as well as to secrete a wide range of biologically active substances that stimulate the processes of reparative regeneration of the organ. Progress in biotechnology and accumulation of knowledge about the mechanisms of functioning of isolated human cells allowed in the middle of the 20 century to begin intensive introduction into clinical practice of cell-tissue transplantation.

Transplantation of stem cells is a unique operation, introducing into the patient's body cells, which are a cell suspension of the primary culture of cryopreserved stem cells derived from the e.g. aborted material (fetus) of 16-21 weeks' gestation. However other sources of stem cells, in particular hepatocytes, are also applicable. The present invention is not directed to or restricted by the source of the stem cells but to the provision of a and a dosage regime for treatment in liver diseases.

The procedure of stem cell transplantation is carried out 6-10 times at intervals of 1-2-3-6 or more months, depending on the severity of the disease and functional disorders of the organ, after the provision of informed consent by the patient. They do not have the ability to unlimited reproduction and differentiation into any kind of specialized cells. The cells have already started differentiation and, therefore, each of them, firstly, can undergo only a limited number of divisions, and, secondly, give rise not to any, but rather certain types of specialized cells. This fact makes their clinical use safer.

### Innovation of the harvesting technique of stem cells for preservation and transplantation.

The human liver after induced miscarriage at termination of pregnancy on social indications (according to the order of the Ministry of Health of the Republic of Kazakhstan Nº KP 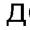 CM-122/2020 from 09.10.2020) with gestational age of 16-20 weeks was used as donor material according to one embodiment. The fetal weight at anthropometry was 200.0-350.0 g and corresponded to the WHO criteria for live birth and stillbirth.

The donor material was taken in the city obstetric centers in accordance with the laws of the Republic of Kazakhstan:
- The final decision to terminate a pregnancy must be made by the woman before the possible use of the tissue is contemplated;
- The woman must not know the recipient of the aborted material in order to rule out an attempted abortion for the purpose of treating a relative or for profit;
- The abortion technique must be based on the safety of the woman.

Harvesting of material for stem cell transplantation (hepatocytes) was performed in accordance with the law on transplantation, with observance of ethical and legal rules and regulations. Maternal and blood was taken for testing (for HIV, RW, etc.) at the time of collection according to the norms. Contraindications to obtaining biological material from the liver were: maternal history of viral hepatitis, tuberculosis, syphilis, HIV, gonorrhea or cytomegalovirus infection. Any possibility of infection of the recipient was excluded.

Material was collected and prepared by the staff team in a special isolation ward in compliance with appropriate standard procedures on sterility. The sterility of the prepared material was controlled with using a standard microbiological method.

### The Method of long-term cryopreservation of hepatocytes

The method of stem cell cryopreservation includes homogenization of the liver, obtaining a washed suspension of primary cell culture, whereby the suspension of washed hepatocytes is placed into the Hanks medium, sterile glycerol solution is added as cryoprotectant in the ratio hepatocytes in Hanks medium - sterile glycerol - 10: 1, cool the resulting mixture to +4 °C for 30 minutes, then freeze to -25 °C at a rate of 0.5 °C/min for 1 hour, incubate at -25 °C for 30 minutes, then freeze to -85 °C at a rate of 0.5 °C/min for two hours, then continue to store the resulting cells in a low-temperature refrigerator at -86 degrees. The method provides long-term cryopreservation of stem cells and makes it possible to create a bank of stem cells (hepatocytes) with a storage period of up to 1 year.

Implementation of the research results has allowed to develop methods for long-term maintenance of viability of human stem cells (hepatocytes) under hypothermia, as well as a path of intravenous injection is introduced into clinical practice of the transplantology department of the West-Kazakhstan Medicine University (WKMU) of the medical center named after Marat Ospanov.

The technical result of the invention is an increase in treatment efficiency, prevention of complications and improvement of the quality of life of patients with chronic diffuse liver lesions.

The disadvantage of other methods of introducing (selective, interventional) cells is surgery, traumatism, high risk of bleeding, hemorrhagic syndrome, as well as contraindications in the form of marked hemostasis disorders in the consequence of liver failure.

Innovation of the method of chronic diffuse liver disease treatment consists in intravenous transplantation of the liver stem cells through the venous cubital catheter allowing to avoid complications from invasion and to exclude iatrogeny in the postoperative period and to refuse immunosuppressive therapy and to consider the stem cell transplantation technique as a new perspective of improving compensation possibilities, life quality and complications prevention in this category of patients. The technical result of the invention is an increase in treatment efficacy, prevention of complications and improvement of well-being of patients with chronic diffuse liver lesions with observance of accompanying therapy leads to an increase in survival rate. This is achieved by the fact that in the claimed method of treatment of chronic diffuse liver damage including intravenous transplantation of liver stem cells, according to the invention, in addition to standard hepatoprotective therapy, a suspension of liver stem cells is intravenously injected in the volume of 6-9×10⁶ and diluted to 20 ml of 0.9% NaCl solution.

When assessing the clinical effect there are specific and nonspecific mechanism of action of stem cells. The specific mechanism of action is a replacement cell therapy - the introduction of living embryonic cells that live in the body for some time and compensate for specific defects in the functions of organs and tissues of the recipient. Tellingly, transplantation of 1-3% hepatocytes compensates for almost any metabolic defect in the liver. The injected donor cells multiply and form stable growths in the recipient's body. biotechnology focuses not on the treatment of old, irreversibly altered cells, but on their timely replacement with new cells by transplanting a pool of donor cells, or by stimulating the recipient's own regeneration reserve. The nonspecific mechanism of action manifests itself in stimulation of regenerative processes due to stadiospecific proteins, peptides (growth factors, cytokines, tissue hormones).
cells and tissues have a number of properties in the recipient's body: they are capable of undergoing changes and differentiation in response to environmental stimuli, in accordance with the embedded genetic information. High content of blast cells causes growth, migration and possibility to form intercellular contacts; they produce and contain a large number of various bioactive substances, such as growth factors, capable of ensuring their survival and/or stimulation of regeneration of damaged tissues of the recipient. In addition, they contain specific proteins and peptides, including alpha-fetoprotein, antioxidants and reactive oxygen species interceptors, adaptogens, anti-inflammatory bacteriostatic compounds, peptides stimulating immunocompetent cells, etc. Foetal cells do not cause immune rejection reaction, as in the 1st and 2nd trimesters of gestation histocompatibility proteins of the 1st and 2nd classes are not yet expressed, they are able to use an evolutionary older energy pathway - glycolysis, so they are more resistant to hypoxia.

The inventive method may be constituted by a method of chronic diffuse liver disease treatment including standard hepatoprotective therapy and transplantation of stem cells (hepatocytes) differing in that liver stem cell suspension is injected intravenously in the volume of 5 ml of cell suspension (6-9×10⁶) and diluted to 20 ml of 0.9% NaCl solution for intravenous administration.

### Method Implementation

Suspension of stem cells is obtained in the laboratory of transplantology of the medical center named after Marat Ospanov. The laboratory is equipped with all up-to-date necessary equipment. After thawing in a water bath according to the accepted method at 37°C, a single cell culture is washed from the cryoprotectant in 199 nutrient medium, and then in Sol. NaCl 0.9%, in 5 ml of cell suspension (6-9×10⁶) diluted in Sol. NaCl 0.9% to 20ml, and administered intravenously. At microscopy before transplantation, after counting on a Goryaev chamber, the percentage of viable cells was 75-80%, and the remaining mass of cells after apoptosis.

To date, many transplants of stem cells (hepatocytes) have been performed and proven effective in improving general condition and blood counts with a new intravenous method of administration in chronic diffuse liver lesions. The modified method of cell therapy for intravenous transplantation of stem cells to decompensated patients with chronic diffuse liver lesions improves outcomes.

### Example 1.

Patient B.K., born in 1955 was repeatedly hospitalized in the department of transplantology of the medical center named after Marat Ospanov.

Clinical diagnosis: liver cirrhosis as an outcome of chronic viral hepatitis C, Child-Pugh severity class B 8 points, decompensation stage. Complications: (K76.6) Portal hypertension. Chronic liver failure. Associated diagnosis: (K80.2) Gallbladder stones without cholecystitis. (B18.2) Chronic viral hepatitis C.

Past medical history: she has had cirrhosis of the liver since 2017, is registered at the place of residence, constantly takes Aldaron 100mg 1 tablet in the morning on an empty stomach, ursosan 250mg 2 capsules 2 times a day. Last hospital stay at the Emergency Department from 11.05 till 22.05.2020 for decompensation, the patient was discharged with improvement. The patient was sent by the portal to the National Scientific Medical Center in Nur-Sultan for stem cell transplantation, but due to the epidemiological situation with coronavirus she was sent back. For the last few weeks she noted increase in abdomen volume, jaundice of sclerae, increasing weakness, fatigability, did not consult doctors, at home she took furosemide, Essenziale, intravenous heptral, her condition never improved. On 20/01/21, she was taken by ambulance to the ASC because of internal bleeding. A surgeon examined her in the emergency room and admitted her to the surgical department. The examination revealed a positive marker for hepatitis C. The patient was administered symptomatic therapy, with improvement. 27.01.21┌. Hospitalized to address the issue of stem cell transplantation (hepatocytes).

Treatment: Diet: 5. Regimen: 2b - ward. Prescribed medications: Ursodex (250 mg, Capsules) (500mg Orally) (1 p/d. 3 d.) Veroshpiron (100 mg, Capsules) (100mg Orally) (1 p/d. 3 d.) Allergopress (1 ml, 2% Solution) (1 ml Intramuscular) (1 p/d. 1 day) Prednisolone (1 mL, 30 mg/mL Solution) (3 mL Intravenously) (1 p/d. 1 day) Ketotop^{®} (2 mL, 100 mg/2 mL Solution) (2 mL Intramuscularly) (1 p/d. 1 day).

Intravenous transplantation of stem cells in the volume of 6-9×10⁶ diluted to 20 ml of 0.9% NaCl solution was performed. Discharged in satisfactory condition under the supervision of a hepatologist.

The lower table shows the dynamic changes of indicators, with transplants there is a decrease in bilirubin, normalization of coagulogram indicators, reduction of clinical symptoms of liver failure.

**Examination and results of stem cell transplantation:**

| Transplantation dates | 28.01.2021 | 05.03.2021 | 07.04.2021 | 21.05.2021 | 02.07.2021 |
|---|---|---|---|---|---|
| Parameters | Nº1 | Nº2 | Nº3 | Nº4 | Nº5 |
| Complaints on admission: | abdominal enlargement, jaundice of sclera, rapid fatigability, reduced appetite, marked general weakness | abdominal enlargement, jaundice of sclera, rapid fatigability, reduced appetite, marked general weakness | rapid fatigability, general weakness, yellowing of skin and sclerae | rapid fatigability, general weakness, yellowing of skin and sclerae | moderate general weakness |
| Total bilirubin, µmol/l | 121,3 | 61,6 | 48,8 | 46 | 35,17 |
| Direct bilirubin, µmol/l | 56,5 | 36,4 | 37,6 | 24,9 | 23,6 |
| Total protein, g/l | 54 | 69 | 65,4 | 73,2 | 63,5 |
| Albumin, g/l | | | | 32 | 37,3 |
| Prothrombin time (PT), sec | 23,26 | 20 | 16,96 | 16,8 | 14,17 |
| Prothrombin index, % | 49,7 | 59,9 | 73,4 | 74,2 | 96,3 |
| Hb, g/l | 79 | 76 | 66 | 68 | 66 |
| White blood cells (WBC)10^9/ 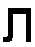 | 9,3 | 5,3 | 3,8 | 4,1 | 3,2 |
| Platelets (PLT) 10^9/ 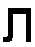 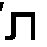 | 98 | 125 | 186 | 121 | 96 |

Ultrasound of the hepatobiliopancreatic area (liver, gallbladder, pancreas, spleen) (06/04/2021) Conclusion: Hepatosplenomegaly. Diffuse cirrhotic changes of liver parenchyma (cirrhosis).

Chronic calculous cholecystitis. Ascites.

Ultrasound of hepatobiliopancreatic area (liver, gallbladder, pancreas, spleen) (20.05.2021) Conclusion: moderate ascites. Hepatosplenomegaly. Diffuse changes of the liver parenchyma. Signs of portal hypertension. Chronic calculous cholecystitis. "Disconnected gallbladder."

Recommendations on discharge: 1. 2. Diet, feeding regime. 3. Ursosan 250 mg 2 drops at night for 6 months. 4. Spironolactone 100mg (veroshpiron, aldaron) in the morning constantly. 5. Dufalac 20 ml 2 times a day for 10 days a month. 6. Bisoprolol 2.5 mg at 10 a.m. long-term (to reduce portal hypertension). 7. Monitor diuresis, body weight, and abdominal volume.

### Example 2.

Patient: I.A., born in 1962 was on in-patient treatment in the department of transplantology of the medical center named after Marat Ospanov in 2021, several times for transplantation treatment.

Clinical diagnosis: (K74.6) Other and unspecified liver cirrhosis.

From the anamnesis: He considers himself a patient since 02.2020, when, after drinking alcohol, signs of liver failure appeared, due to which he was hospitalized at the WKMU with the diagnosis: Toxic liver damage. On examination, liver cirrhosis was diagnosed for the first time. He was observed on an outpatient basis, constantly receiving hepatoprotectors. Periodically, in case of decompensation of cirrhosis, he is treated in the hospital. Since September, 2020, the patient has been undergoing HSCT, he is noticing an improvement.

Treatment regimen: 5 Regimen: 2b - ward. Prescribed medications: Veroshpiron (100 mg, Capsules) (100mg Orally) (1 p/d. 3 d.) Trigrim (10 mg, Tablets) (10mg Orally) (1 p/d. 3 d.) Ursodex (250 mg, Capsules) (500mg Orally) (1 p/d. 3 d.) Amlodipine (5 mg, Tablets) (2.5mg Orally) (1 p/d 3 d.) Omez^{®} (20 mg, Capsules) (20mg Orally) (2 p/d 2 d.) Omez^{®} (20 mg, Capsules) (20mg Orally) (1 p/d 1 d.)
Intravenous transplantation of stem cells in the volume of 6-9×10⁶ diluted to 20 ml of 0.9% NaCl solution was performed. Discharged in satisfactory condition under the supervision of a hepatologist.

The lower table shows dynamic changes in the indicators, at the next transplants, a decrease in bilirubin, stabilization of coagulogram and platelet counts, reduction of clinical symptoms of liver failure are noted.

**Examination and results of stem cell transplantation:**

| Transplant dates | 07.04.2021 | 03.06.2021 | 23.09.2021 | 21.12.2021 |
|---|---|---|---|---|
| Parameters | Nº2 | Nº3 | Nº4 | Nº5 |
| Complaints on admission: | rapid fatigability, general weakness, decreased appetite, swelling of the lower extrem ities | rapid fatigability, general weakness, decreased appetite, pastosity of the lower extrem ities | no | no |
| Total bilirubin, µmol/l | 36,1 | 33,9 | 36,1 | 24,3 |
| Direct bilirubin, µmol/l | 14 | 12,9 | 11,77 | 11,3 |
| Total protein g/l | 64,1 | 65 | 60,45 | 61,6 |
| Albumin, g/l | 32 | | | 34,2 |
| Prothrombin time (PT), sec | 15,28 | 15,65 | 16,38 | 16,24 |
| Prothrombin index, % | 83,5 | 88,4 | 84,4 | 85,2 |
| Hb, g/l | 113 | 123 | 117 | 115 |
| White blood cells (WBC) 10^9/ 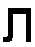 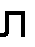 | 5 | 4 | 4,55 | 6,3 |
| Platelets (PLT) 10^9/ 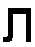 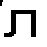 | 136 | 145 | 144 | 181 |

Ultrasonic examination of the hepatobiliopancreatic area (liver, gallbladder, pancreas, spleen) (06.04.2021) Conclusion: Diffuse cirrhotic changes in the liver parenchyma (cirrhosis). Chronic calculous cholecystitis. Diffuse changes of pancreatic parenchyma. Splenomegaly.

Ultrasound of hepatobiliopancreatic area (liver, gallbladder, pancreas, spleen) (12/21/2021) Conclusion: Cirrhosis echo pattern of the liver. Chronic calculous cholecystitis. Chronic pancreatitis. Slight enlargement of the spleen area.

### List of references:

1. Asrani S.K., Devarbhavi H., Eaton J., Kamath P.S. Burden of liver diseases in the world. J. Hepatol. 2019;70:151-171. doi: 10.1016/j.jhep.2018.09.014.
2. L. Edgar, T. Pu, B. Porter, J.M. Aziz et al. Regenerative medicine, organ bioengineering and transplatation British Journal of Surgery, Volume 107, Issue 7, June 2020, Pages 793-800, https://doi.org/10.1002/bjs.11686.
3. stem cell transplantation. RCHD (Republican Center for Health Care Development of the Ministry of Health care of the Republic of Kazakhstan). Version: Clinical Protocols of MoHC of RoK-2015.
4. Tuganbekova SK, Rakhmetova VS, Kutmanova AZ, Kuzembekova KU, Shaymardanova GM, Askarov MB, Saparbaev SS, Popova NV, Dosataeva G.S. Regenerative bases of cell technologies in liver cirrhosis: monograph Astana, 2015.// 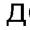 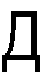 616.3(035.3), 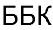 K: 54.13, ISBN 978-601-244-277-9.
5. Tuganbekova S.K., Rakhmetova V.S., Kutmanova A.Z., Kuzembekova K.U., Saparbaev S.S., Popova N.V., Dosataeva G.S. Cell therapy in the complex treatment of liver cirrhosis in the outcome of viral hepatitis: guidelines Astana, 2015.// 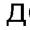 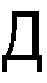 616.9, 66K: 55.1, ISBN 978-601-305-107-9.
6. Saparbayev S.S., Alzhanova D.S., Comparative assessment of clinical and neuroimaging features and quality of life in patients with syringomyelia on the background of cell therapy: monograph Nur-Sultan, 2020.// 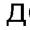 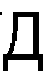 616, 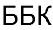 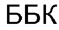 : 53.5, ISBN 978-601-80818-5-9.
7. Saparbayev S.S., Taubaldieva J.S., Rustemova K.R., Experience of cell therapy in hypothyroidism and clinical and functional characteristics in thyrocyte transplantation: Nur-Sultan monograph, 2020.// 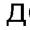 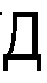 616, 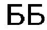 : 51.1, ISBN 978-601-80818-2-8.
8. Saparbayev S.S., Bukeyeva J.K., Akpolatova G.M., Tarzhanova D.S., Effect of mediator substances of cells on the course of acute and chronic hepatitis in rats: Nur-Sultan monograph, 2020.// 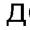 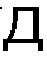 616, 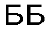 : 54.1, ISBN 978-601-80818-7-3.
9. Saparbayev S.S., Kayupov B.A., Shakenov A.D., Application of nephrocytes in surgical treatment of chronic renal insufficiency: Nur-Sultan monograph, 2020.// 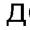 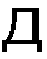 61.6, 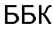 : 54.5, ISBN 978-601-80818-3-5.
10. Saparbayev S.S., Tarzhanova D.Sh., Effect of "mediators" of liver tissue on the course of experimental diabetes mellitus: Nur-Sultan monograph, 2020.// 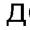 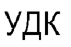 616, 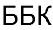 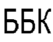 : 54.1, ISBN 978-601-80817-8-1.
11. Saparbayev S.S., Akpolatova G.M., Effect of "mediator substances" of liver tissue on changes in the adaptive reactions of the body under stress caused by immobilization and hypoxia (experimental study): monograph Nur-Sultan, 2020.// 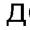 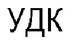 616, 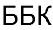 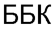 : 54.1, ISBN 978-601-80817-9-8.
12. Saparbayev S.S., Kushenova S.J., Cell mediators in complex intensive therapy of postoperative DIC syndrome: Nur-Sultan monograph, 2020.// 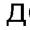 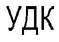 616, 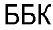 : 54.10, ISBN 978-601-80817-7-4.
13. Saparbayev S.S., Konakbay B.K., Shakenov A.D., The role of cell mediators in complex treatment of endogenous intoxication syndrome in patients with complicated forms of echinococcosis: Nur-Sultan monograph, 2020.// 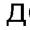 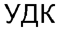 616, 66K: 54.19, ISBN 978-601-80817-6-7-7.
14. Saparbayev S.S., Regenerative medicine and cellular technologies in practical medicine: monograph Astana, 2020.// 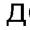 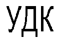 616-003, 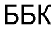 : 54.1, ISBN 978-601-80817-3-6.
15. Saparbayev S.S., Syzdykova B.R., Effect of neurocyte transplantation on clinical and immunological parameters in patients with multiple sclerosis: monograph Nur-Sultan, 2020.// 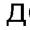 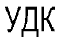 616, 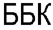 : 53.3, ISBN 978-601-80818-4-2.
16. Saparbayev S.S., Correction of condition in clinical practice in complex treatment of intoxication and DIC syndrome by mediators of hepatocytes: monograph Nur-Sultan, 2020.// 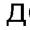 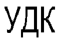 616, 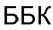 : 54.1, ISBN 978-601-80818-6-6.
17. Baigenjin A.K., Kayupov B.A., Saparbaev S.S., Askarov M.B., Doskaliev Zh.A., Akhaeva A.A., Zhakupova A.H., Sembaev K.D., Title: Method for producing lyophilizate from human hepatocytes: Patent (PCT) November 15, 2012 (15/11/2012) W P O I R S T// WO 2012/154016 A 1, PCT/KZ201 1/000009.

## Claims

1. Dosage regime of a stem cell, preferably hepatocytes, suspension for the treatment of chronic diffuse liver disease
**characterized by**
a dosage concentration that corresponds or equals to 5 ml of cell suspension with a concentration of 6-9×10⁶ stem cells per mL which is diluted to 20 ml of 0.9% NaCl solution for administration.

2. Dosage regime according to claim 1
**characterized in that**
the suspension is provided as a volume of 5 ml of cell suspension with a concentration of 6-9×10⁶ stem cells per mL which is diluted to 20 ml of 0.9% NaCl solution for administration.

3. Stem cell suspension, preferably of hepatocytes, for use in the treatment of chronic diffuse liver disease.

4. Stem cell suspension according to claim 3
**characterized in that**
the suspension is provided in NaCl solution.

5. Stem cell suspension according to claim 3 or 4
**characterized in that**
the stem cells are fetal stem cells.
